# EUROPEAN PATENT SPECIFICATION

(11) **EP 2 080 497 B1**
(45) Date of publication and mention of the grant of the patent: **08.07.2015**
(21) Application number: 07831304.6
(22) Date of filing: 06.11.2007
(51) Int. Cl.: A61F 13/49, A61F 5/44, A61F 13/15, A61F 13/511, A61F 13/513

(54) **ABSORPTIVE ARTICLE**
SAUGFÄHIGER ARTIKEL
ARTICLE ABSORBANT

(30) Priority: 06.11.2006 JP 2006300261
(43) Date of publication of application: 22.07.2009
(73) Proprietor: Uni-Charm Corporation, Ehime 799-0111 (JP)
(72) Inventor: SUGITO, Tomoko, Kagawa 769-1602 (JP); KIKUCHI, Kyo, Kagawa 769-1602 (JP); SHIMIZU, Jyoji, Kagawa 769-1602 (JP); NAKAJIMA, Osamu, Kagawa 769-1602 (JP)
(74) Representative: Murgatroyd, Susan Elizabeth
(86) International application number: PCT/JP2007/071572
(87) International publication number: WO 2008/056675

(56) References cited:
- EP-A1- 1 133 962
- EP-A2- 0 997 124
- WO-A1-02/085274
- WO-A1-2005/013874
- JP-A- 10 094 558
- JP-A- 2001 231 815
- JP-A- 2001 328 191
- JP-A- 2004 181 087

## Description

### TECHNICAL FIELD

The present invention relates to an absorbent article suitably applicable to a top sheet (surface material) for a disposable diaper, a sanitary napkin, a panty liner, an incontinence pad, and the like.

### BACKGROUND ART

Heretofore, an absorbent article has been known in which crosswise emboss lines are formed in both end portions of a top sheet in a longitudinal direction, and in which lengthwise emboss lines are formed in an approximately center portion thereof (see Patent Document 1) . In such an absorbent article, the lengthwise emboss lines act to spread body fluid in the longitudinal direction, thereby preventing the body fluid from spreading in the cross direction. The crosswise emboss lines act to spread the body fluid in the cross direction, thereby preventing the body fluid from spreading in the longitudinal direction. In this way, the body fluid is prevented from leaking out from the end portions of the absorbent article in both the cross direction and the longitudinal direction.

However, since a conventional absorbent article is configured to prevent the body fluid from leaking out by controlling the spreading direction of the body fluid on the surface of its top sheet as described above, the absorbent article fails to keep the spread area of body fluid on the surface of the top sheet small when the body fluid has high viscosity like water-like faeces or menstrual blood, and accordingly does not easily pass through the top sheet.

EP 0997124 A2 discloses a disposable body fluids absorbent article including a topsheet made of non-woven fabric and being formed with a plurality of pleats extending substantially in one direction. Crests and troughs of the pleats each extends along the longitudinal direction and the crests and the troughs are provided alternately in the crossing direction. Also, a fiber density of a wall defined between the crest and the trough is lower than the fiber density of the crest and the trough.

EP 1133962 A1 discloses a porous sheet made of non-woven fabric and having a corrugated or wavy configuration with crests and troughs. Each of the crests and the troughs extends along the crossing direction and the crests and the troughs are provided alternately in the longitudinal direction. Also, the fiber density of a wall defined between the crest and the trough is lower than the fiber density of the crest and the trough.

The present invention has been made to solve the aforementioned problem, and aims to provide an absorbent article capable of controlling the spread area of body fluid on the surface sheet thereof.
Patent Document 1: Japanese Patent Application Publication No. 2004-298271.

### DISCLOSURE OF THE INVENTION

According to the present invention, an absorbent article comprises a surface sheet made of a liquid permeable fiber nonwoven fabric and shaped in a waveform; a liquid impermeable back surface sheet; and an absorber interposed between the surface sheet and the back surface sheet; wherein the waveform is continuously formed in the longitudinal direction of the surface sheet, a top portion and a bottom portion of the waveform each extends in a direction crossing the longitudinal direction of the surface sheet, and a fiber density in a wall area between a top area and a bottom area of the waveform is lower than a fiber density in any one of the top area and the bottom area; characterized in that: fibers constituting the surface sheet are more oriented in a longitudinal direction of the surface sheet than in a cross direction thereof; the waveform is continuously formed in the longitudinal direction in multiple shape forming regions of the surface sheet, the multiple shape forming regions each extending in the longitudinal direction and being aligned in the direction crossing the longitudinal direction; a non-shape forming region is formed between each adjacent two of the shape forming regions, the non-shape forming regions also extending in the longitudinal direction; and the shape forming regions are located in a position shifted to the side of a back waistband region.

With use of the absorbent article according to the present invention, components in body fluid that easily pass through the surface sheet mainly pass through the surface sheet through the wall area while only high viscosity components thereof remain on the surface sheet. This increases the viscosity of the remaining body fluid, whereby the spread area of the body fluid on the surface sheet can be kept small.

### BRIEF DESCRIPTION OF THE DRAWINGS

[Fig. 1] Fig. 1 is a schematic view showing an overall configuration of a disposable diaper illustrated as an embodiment of the present invention.
[Fig. 2] Fig. 2 is a schematic view showing a cross section configuration along a longitudinal-direction of a top sheet illustrated as the embodiment of the present invention.
[Fig. 3] Fig. 3 is an upper surface view showing a configuration of the top sheet illustrated as the embodiment of the present invention.
[Fig. 4] Fig. 4 is a schematic view showing orientation of fibers in a shape forming region shown in Fig. 3.
[Fig. 5] Fig. 5 is a schematic view for explaining a method of manufacturing the top sheet illustrated as the embodiment of the present invention.

### BEST MODE FOR CARRYING OUT THE INVENTION

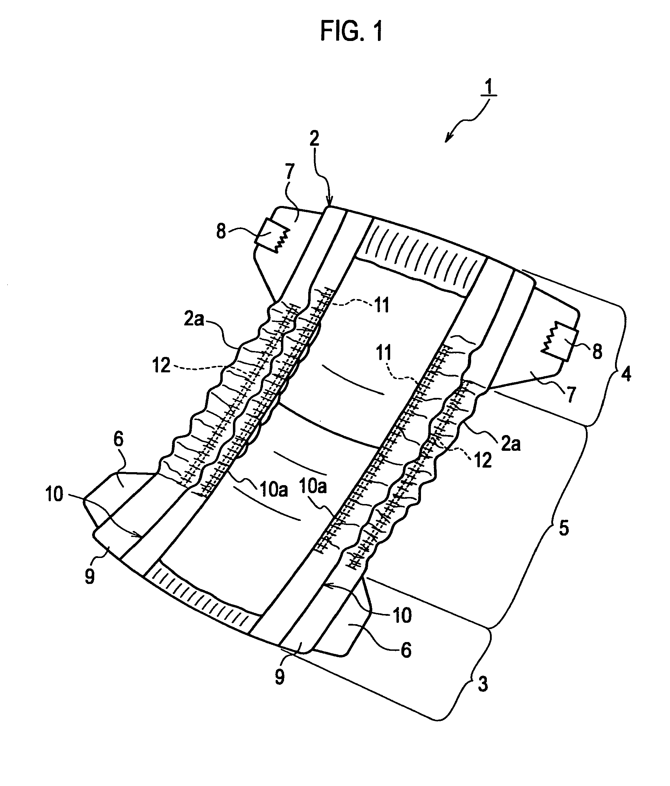

A water absorbent article according to the present invention is applicable to a disposal diaper as shown in Fig. 1, for example. Hereinafter, a configuration of a disposable diaper illustrated as an embodiment of the present invention will be described with reference to the drawings. It should be noted that an absorbent article according to the present -invention is not limited to this embodiment, and is also applicable to a top sheet for, for example, a sanitary napkin, a panty liner, an incontinence pad or the like.

### [Overall Configuration of Disposable Diaper]

As shown in Fig. 1, a disposable diaper 1 described as the embodiment of the present invention includes a water absorbent article 2, and is configured of a front waistband region 3 and a back waistband region 4 in front and back ends thereof in a longitudinal direction and a crotch region 5 located between the front waistband region 3 and the back waistband region 4. The front waistband region 3 includes wing portions 6 and the back waistband region 4 includes wing portions 7. The wing portions 6 and 7 each extend in a lateral side direction of the disposable diaper 1. When the disposable diaper 1 is worn, the wing portions 6, 7 overlap each other with the wing portions 6 placed on the inner side and are joined with each other with a tape fastener 8.

Cuffs 10 are formed at both cross direction side portions of the water absorbent article 2 so as to extend in a longitudinal direction along cross direction side edges 2a of the water absorbent article 2. Each of the cuffs 10 is formed by folding back a sheet member 9 that forms a surface layer over the cross direction side portions of the water absorbent article 2 and the wing portions 6. At a longitudinal direction center portion of the water absorbent article 2, this cuff 10 is folded and thus formed to project from the surface of the water absorbent article 2 at an approximately constant range (height), and a string-like elastic member 11 is attached to a tip edge 10a of each of the cuffs 10 in an elastic state.

The attaching of the elastic members 11 causes the tip edges 10a of the cuffs 10 to shrink in the longitudinal direction, thereby forming gathers in the cuffs 10, and also causes a force of pulling both longitudinal direction sides of the water absorbent article 2 toward the center side thereof to act through the cuffs 10. Thereby, the water absorbent article 2 is provided with tendencies to be bent in the longitudinal direction with the side of a top sheet 21, which will be described later, facing inward, and to have the cuffs 10 stand like walls at the longitudinal direction center portion of the water absorbent article 2.

At the longitudinal direction center portion of the water absorbent article 2, a string-like elastic member 12 is provided in a location shifted from a fixed edge of each of the cuffs 12 outwardly in the cross direction of the water absorbent article 2 (toward the cross direction side edge 2a). The elastic member 12 is provided in an elastic state so as to extend along the cross direction side edge 2a and the fixed edge. The attaching of this elastic member 12 causes the cross direction side portion of the water absorbent article 2 to form gathers.

### [Configuration of Water Absorbent Article]

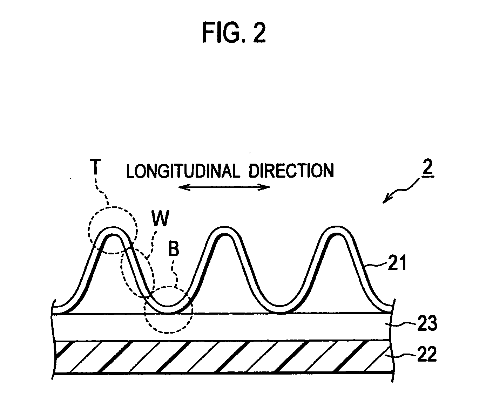

As shown in Fig. 2, the water absorbent article 2 includes: the liquid permeable top sheet (surface sheet) 21 formed of a fiber nonwoven fabric; a liquid impermeable back sheet (back surface sheet) 22 formed of a plastic film, a composite material of a plastic film and a nonwoven fabric, or the like; and a liquid absorbent core (absorber) 23 interposed between the top sheet 21 and the back sheet 22 and joined to at least one of the top sheet 21 and the back sheet 22.

A material usable for the liquid absorbent core 23 is a mixture of particulate or fibrous super absorbent polymer and fluff pulp, a mixture of particulate or fibrous super absorbent polymer, fluff pulp and thermoplastic synthetic resin fiber, or other similar mixtures, the mixtures each being compressed in a desired thickness. In addition, the entire liquid absorbent core 23 is desirably covered with tissue paper in order to prevent deformation thereof and dropping-off of the polymer particles. As the polymer particles, a starch type, a cellulose type, or a synthetic polymer type is usable.

### [Configuration of Top Sheet]

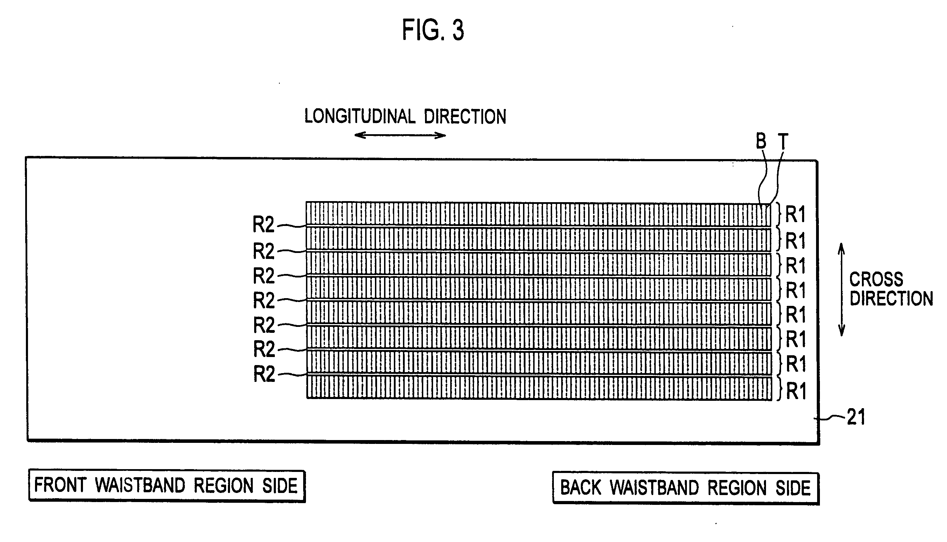

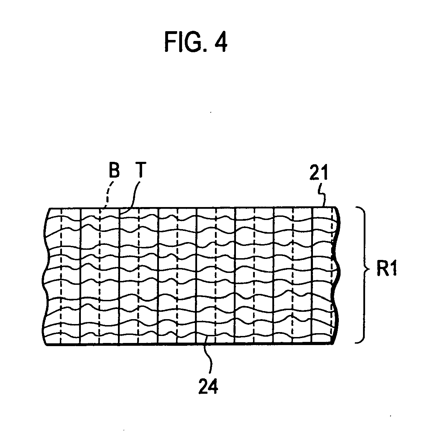

As shown in Fig. 3, in the top sheet 21, multiple shape forming regions R1 are each formed to extend in the longitudinal direction of the absorbent article, and are arranged in a direction (cross direction) crossing the longitudinal direction of the absorbent article. In each of the shape forming regions R1, a waveform composed of a top area T, a bottom area B, and a wall area W between the top area T and the bottom area B is continuously formed in the longitudinal direction, as shown in Fig. 2. In addition, as shown in Fig. 4, fibers 24 constituting the top sheet 21 are more oriented in the longitudinal direction of the top sheet 21 than in the cross direction. The fibers 24 are formed, for example, by fusion bonding of the fibers in the top area T or the bottom area B, so that the fiber density in the wall area W is lower than the fiber density of the top area T or the bottom area B. Instead, the fibers 24 may be formed by cutting off the fibers or bonding portions of the fibers in the wall area W, so that the fiber density in the wall area W is lower than the fiber density of the top area T or the bottom area B. Otherwise, the fibers 24 may be formed by drawing the fibers in the wall area W to make the fiber diameter in the wall area W smaller (thinner) than the fiber diameter in the top area T and the bottom area B, so that the fiber density in the wall area W is lower than the fiber density of the top area T or the bottom area B. Specifically, the fiber densities in the top area T and the bottom area B are each set within a range of 0.05 [g/cm³] to 0.2 [g/cm³] inclusive, while the fiber density in the wall area W is set within a range of 0. 005 [g/cm³] to 0.15 [g/cm³] inclusive.

When the waveform composed of the top area T, the bottom area B, and the wall area W is continuously formed in the longitudinal direction of the top sheet 21 as described above, body fluid is more likely to spread along the bottom areas B, whereby the spread direction of the body fluid can be controlled. In addition, when the fiber density in the wall area W is made lower than the fiber density in the top area T or the bottom area B, components of the body fluid that easily pass through the top sheet 21 mainly pass through the top sheet 21 through the wall areas W and only high viscosity components are left on the surface of the top sheet 21. Thus, the viscosity of the body fluid is so high that the spread area of the body fluid can be kept small. As a result, this leads to a reduction in the area where the body fluid adheres to the skin, and prevents the body fluid from leaking out from the absorbent article. Incidentally, when the fibers in the top area T and the bottom area B are fusion bonded, the fibers are fixed in a manner crossing the orientation direction of the fibers, and the fibers are surely oriented and extend between the adjacent fusion bonded portions.

As shown in Fig. 3, the shape forming regions R1 are located in a position shifted to the side of the back waistband region 4. The top sheet allows low viscosity body fluid such as urine to pass therethrough, but hardly allows high viscosity body fluid such as water-like feces to pass therethrough. In addition, the high viscosity body fluid is highly likely to spread on the back side of the absorbent article. Moreover, in the case where the top sheet is shaped in the waveform as described above, the irritation of the skin increases. Thus, it is desirable to provide the shape forming regions R1 only to a minimum necessary extent. For these reasons, by locating the shape forming regions R1 in the position shifted to the side of the back waistband region 4, the spread area of high viscosity body fluid can be kept small and the irritation of the skin can be minimized.

A non-shape forming region R2 is formed between each adjacent two of the shape forming regions R1. The non-shape forming region R2 extends in the longitudinal direction and has a cross direction dimension of 0.2 [mm] to 0.3 [mm] inclusive. The formation of the non-shape forming region R2 between the shape forming regions R1 prevents a situation where the body fluid spreads because the wall areas W in the shape forming regions R1 are accordion-folded. In addition, this configuration allows the tension of the top sheet 21 to be maintained at the time of manufacturing the top sheet 21, thereby preventing the top sheet 21 from becoming loose and crinkled.

The top area T and the bottom area B each have dimensions within ranges of 0.2 [mm] to 3 [mm] inclusive in the longitudinal direction and of 3 [mm] to 100 [mm] inclusive in the cross direction, and the wall area W has dimensions within ranges of 0.3 [mm] to 5 [mm] inclusive in the longitudinal direction and of 3 [mm] to 100 [mm] inclusive in the cross direction. In addition, the height dimension of the waveform from the bottom portion to the top portion is within a range of 0.3 [mm] to 3 [mm] inclusive.

As described above, the fiber densities in the top area T and the bottom area B are higher than the fiber density in the wall area W. For this reason, when the area ratios of the top area T and the bottom area B are set high, the permeability of body fluid is lowered. To avoid this, it is necessary to decrease the area ratio of the wall area W by decreasing the area ratios of the top area T and the bottom area B. The increase in the area ratio of the wall area W, however, leads to reduction in the strength of the top sheet 21, whereby skin rubbing or the like is more likely to cause tearing of the top sheet 21 or deformation of the waveform. Therefore, the top sheet 21 is desirably formed in the aforementioned dimensions.

### [Top Sheet Manufacturing Method]

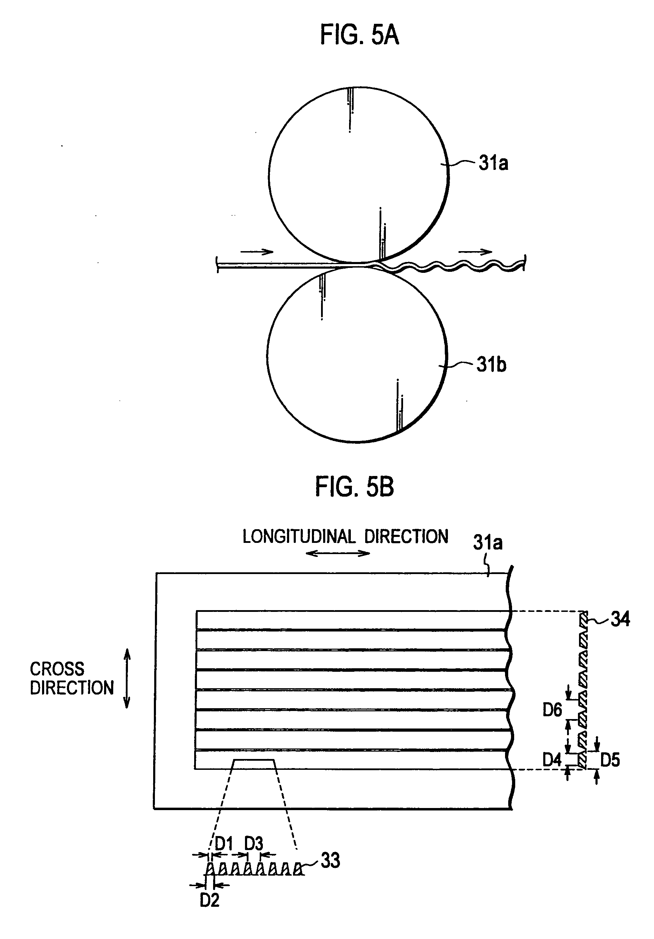

The foregoing waveform of the top sheet 21 can be formed by feeding a fiber nonwoven fabric into between rollers 31a and 31b while applying an appropriate tension to the fiber nonwoven fabric (see Fig. 5(a)). On the surface of each of the rollers 31a and 31b, concave and convex patterns, for example, as shown in Fig. 5(b) are formed. The dimensions D1, D2, and D3 of the upper extremity, the lower extremity, and the length between each adjacent two of the upper extremities of convex portions 33 of multiple trapezoidal shapes formed in the roller 31a in the longitudinal direction and the dimensions D4, D5, and D6 of the upper extremity, the lower extremity, and the length between each adjacent two of the upper extremities of convex portions 34 of multiple trapezoidal shapes formed in the roller 31a in the cross direction are determined appropriately depending on the dimensions of the waveform and shape forming regions to be formed. Note that, an apparatus shown in Fig. 5 is configured so that the concave and convex patterns of the rollers 31a and 31b are meshed with each other, that is, convex portions of the roller 31a are respectively meshed with concave portions of the roller 31b. Thus, the top portions and the bottom portions of the waveform of the top sheet 21 respectively correspond to the concave portions of the roller 31a and the convex portions of the roller 31b. Accordingly, the density of the wall areas in the top sheet 21 can be changed by adjusting the depths and the like of the rollers 31a and 31b as needed.

### [Other Embodiments]

Heretofore, the embodiment to which the present invention made by the present inventors is applied has been described. However, the present invention as defined by the appended claims is not limited by the description and the drawings that are provided according to this embodiment and constitute a part of the disclosure of the present invention. For example, a nonwoven fabric may be formed on the surface, at the side of the liquid absorbent core 23, of the shape forming regions in the top sheet 21. The nonwoven fabric has a density within a range of 0.005 [g/cm³] to 0.10 [g/cm³] inclusive, and thus is more bulky than the top sheet 21. When the liquid absorbent core is covered with a high density sheet, high viscosity body fluid has nowhere to go even once passing through the top sheet, and thus leaks out to remain on or flow over the top sheet 21. For this reason, by providing the bulky nonwoven fabric having the density within the range of 0.005 [g/cm³]to 0.10 [g/cm³] inclusive, high viscosity body fluid flows toward the liquid absorbent core 23 after passing through the top sheet 21, whereby the contact area between the body fluid and the skin can be reduced more easily.

Moreover, in this case, a sheet covering the liquid absorbent core 23 and having a density within a range of 0.05 [g/cm³] to 0.25 [g/cm³] inclusive may be provided between the bulky nonwoven fabric and the liquid absorbent core 23. When the density in the top sheet 21 is lowered, the components of the absorbent core 23 may get out to the surface of the absorbent article 2 through the top sheet 21. The covering of the liquid absorbent core 23 with the sheet having the density within the range of 0.05 [g/cm³] to 0.25 [g/cm³] inclusive prevents the components of the liquid absorbent core 23 from getting out. Furthermore, to add another word or two, it is obvious that all other embodiments, examples, application techniques and the like made based on the foregoing embodiments by those skilled in the art fall under the category of the present invention as defined by the appended claims.

### INDUSTRIAL APPLICABILITY

The present invention is applicable to a top sheet (surface material) for a disposable diaper, a sanitary napkin, a panty liner, an incontinence pad or the like.

## Claims

1. An absorbent article comprising a surface sheet (21) made of a liquid permeable fiber nonwoven fabric and
shaped in a waveform; a liquid impermeable backy surface sheet (22); and an absorber (23) interposed between the surface sheet (21) and the back, surface sheet (22); wherein
the waveform is continuously formed in the longitudinal direction of the surface sheet (22),
a top portion and a bottom portion of the waveform each extends in a direction crossing the longitudinale direction of the surface sheet (21), and
a fiber density in a wall area (W) between a top; area (T) and a bottom area (B) of the waveform is lower than a than a fiber density in any one, of the top area (T) and the bottom area (B);
**characterized in that**:
fibers (24) constituting the surface sheet (21) are more oriented in a longitudinal direction of the surface sheet (21) than in a cross direction thereof;
the waveform is continuously formed in the longitudinal direction in multiple shape forming regions (R1) of the surface sheet (21), the multiple shape forming regions (R1) each extending in the longitudinal direction and being aligned in the direction crossing the longitudinal direction; a non-shape forming region (R2) is formed between each adjacent two of the shape forming regions (R1), the non-shape forming regions (R2) also extending in the longitudinal direction; and
the shape forming regions (R1) are located in a position shifted to the side of a back waistband region.

2. The absorbent article according to claim 1, wherein the fibers (24) in the top area (T) and the bottom area (B) are fusion bonded.

3. The absorbent article according to any one of claims 1 and 2, wherein
the fiber density of each of the top area (T) and the bottom area (B) is within a range of 0.05 [g/cm³] to 0.2 [g/cm³]inclusive, and
the fiber density of the wall area (W) is within a range of 0.005 [g/cm³] to 0.15 [g/cm³] inclusive.

4. The absorbent article according to any one of claims 1 to 3, wherein
the top area (T) and the bottom area (B) each have dimensions within ranges of 0.2 [mm] to 3 [mm] inclusive in the longitudinal direction and of 3 [mm] to 100 [mm] inclusive in the cross direction, the wall area (W) has dimensions within ranges of 0.3 [mm] to 5 [mm] inclusive in the longitudinal direction and of 3 [mm] to 100 [mm] inclusive in the cross direction, and
a height dimension of the waveform from the bottom portion to the top portion is within a range of 0.3 [mm] to 3 [mm] inclusive.

5. The absorbent article according to any one of claims 1 to 4, wherein the fibers (24) in the wall area (W) are drawn, whereby a fiber diameter in the wall area (W) is smaller than fiber diameters in the top area (T) and the bottom area (B).

6. The absorbent article according to any one of claims 1 to 5, wherein in the wall area (W), the fibers (24) or bonding portions of the fibers (24) are cut off.

7. The absorbent article according to any one of claims 1 to 6, wherein the surface sheet (21) includes a non-shape forming region (R2) extending in the longitudinal direction and having a cross direction dimension of 0.2 [mm] to 3 [mm] inclusive.

8. The absorbent article according to any one of claims 1 to 7, comprising a nonwoven fabric that is formed on a surface, at an absorber side, of the shape forming region (R1) in the surface sheet (21) and is more bulky than the surface sheet (21).

9. The absorbent article according to claim 8, wherein the bulky nonwoven fabric has a density within a range of 0.05 [g/cm³] to 0.10 [g/cm³] inclusive.

10. The absorbent article according to any one of claims 8 and 9, comprising a sheet that is disposed between the bulky nonwoven fabric and the absorber (23) and covers the absorber (23), wherein the sheet has a density within a range of 0.05 [g/cm³] to 0.25 [g/cm³] inclusive.

## Patentansprüche

1. Saugfähiger Artikel, umfassend eine Oberflächenbahn (21), die aus einem flüssigkeitsdurchlässigen Faser-Vliesstoff hergestellt und in einer Wellenform geformt ist; eine flüssigkeitsundurchlässige Rückflächenbahn (22); und einen Saugkörper (29), der zwischen der Oberflächenbahn (21) und der Rückflächenbahn (22) angeordnet ist; wobei
die Wellenform kontinuierlich in der Längsrichtung der Oberflächenbahn (21) ausgebildet ist,
ein oberer Teil und ein unterer Teil der Wellenform sich jeweils in einer Richtung erstrecken, die quer zur Längsrichtung der Oberflächenbahn (21) verläuft, und
eine Faserdichte in einem Wandbereich (W) zwischen einem oberen Bereich (T) und einem unteren Bereich (B) der Wellenform niedriger als eine Faserdichte in entweder dem oberen Bereich (T) oder dem unteren Bereich (B) ist;
**dadurch gekennzeichnet, dass**:
Fasern (24), aus denen die Oberflächenbahn (21) besteht, mehr in einer Längsrichtung der Oberflächenbahn (21) als in deren Querrichtung ausgerichtet sind;
die Wellenform kontinuierlich in der Längsrichtung in mehreren Formbildungsbereichen (R1) der Oberflächenbahn (21) ausgebildet ist, wobei die mehreren Formbildungsbereiche (R1) sich jeweils in der Längsrichtung erstrecken und in der Richtung ausgerichtet sind, die quer zur Längsrichtung verläuft;
ein Nicht-Formbildungsbereich (R2) zwischen jeweils zwei nebeneinander liegenden der Formbildungsbereiche (R1) ausgebildet ist, wobei sich die Nicht-Formbildungsbereiche auch in der Längsrichtung erstrecken; und
die Formbildungsbereiche (R1) in einer Position angeordnet sind, die auf die Seite eines hinteren Hüftbandbereichs verschoben ist.

2. Saugfähiger Artikel gemäß Anspruch 1, wobei die Fasern (24) in dem oberen Bereich (T) und dem unteren Bereich (B) verschweißt sind.

3. Saugfähiger Artikel gemäß einem der Ansprüche 1 und 2, wobei
die Faserdichte jeweils des oberen Bereichs (T) und des unteren Bereichs (B) innerhalb eines Bereichs von einschließlich 0,05 g/cm³ bis einschließlich 0,2 g/cm³ ist, und
die Faserdichte des Wandbereichs (W) innerhalb eines Bereichs von einschließlich 0,005 g/cm³ bis einschließlich 0,15 g/cm³ ist.

4. Saugfähiger Artikel gemäß einem der Ansprüche 1 bis 3, wobei
der obere Bereich (T) und der untere Bereich (B) jeweils Abmessungen innerhalb Bereichen von einschließlich 0,2 mm bis einschließlich 3 mm in der Längsrichtung und von einschließlich 3 mm bis einschließlich 100 mm in der Querrichtung haben,
der Wandbereich (W) Abmessungen innerhalb von Bereichen von einschließlich 0,3 mm bis einschließlich 5 mm in der Längsrichtung und von einschließlich 3 mm bis einschließlich 100 mm in der Querrichtung hat, und
eine Höhenabmessung der Wellenform von dem unteren Teil zum oberen Teil innerhalb eines Bereichs von einschließlich 0,3 mm bis einschließlich 3 mm beträgt.

5. Saugfähiger Artikel gemäß einem der Ansprüche 1 bis 4, wobei die Fasern (24) in dem Wandbereich (W) gezogen werden, wobei ein Faserdurchmesser in dem Wandbereich (W) kleiner als Faserdurchmesser in dem oberen Bereich (T) und dem unteren Bereich (B) ist.

6. Saugfähiger Artikel gemäß einem der Ansprüche 1 bis 5, wobei in dem Wandbereich (W) die Fasern (24) oder Verbindungsteile der Fasern (24) abgeschnitten werden.

7. Saugfähiger Artikel gemäß einem der Ansprüche 1 bis 6, wobei die Oberflächenbahn (21) einen Nicht-Formbildungsbereich (R2) aufweist, der sich in der Längsrichtung erstreckt und eine Querrichtungsabmessung von einschließlich 0,2 mm bis einschließlich 3 mm hat.

8. Saugfähiger Artikel gemäß einem der Ansprüche 1 bis 7, umfassend einen Vliesstoff, der auf einer Saugkörperseite auf einer Oberfläche des Formbildungsbereichs (R1) in der Oberflächenbahn (21) ausgebildet ist und voluminöser als die Oberflächenbahn (21) ist.

9. Saugfähiger Artikel gemäß Anspruch 8, wobei der voluminöse Vliesstoff eine Dichte innerhalb eines Bereichs von einschließlich 0,05 g/cm³ bis einschließlich 0,10 g/cm³ hat.

10. Saugfähiger Artikel gemäß einem der Ansprüche 8 und 9, umfassend ein Bahnmaterial, das zwischen dem voluminösen Vliesstoff und dem Saugkörper (23) angeordnet ist und den Saugkörper (23) abdeckt, wobei das Bahnmaterial eine Dichte innerhalb eines Bereichs von einschließlich 0,05 g/cm³ bis einschließlich 0,25 g/cm³ hat.

## Revendications

1. Article absorbant comprenant une feuille de surface (21) réalisée avec un tissu non tissé fibreux perméable au liquide et formé selon une forme d'onde ; une feuille de surface inférieure imperméable au liquide (22) ; et un absorbant (23) intercalé entre la feuille de surface (21) et la feuille de surface inférieure (22) ; dans lequel
la forme d'onde est formée de manière continue dans la direction longitudinale de la feuille de surface (21),
une partie supérieure et une partie inférieure de la forme d'onde s'étendent chacune dans une direction croisant la direction longitudinale de la feuille de surface (21), et
une densité de fibre dans une zone de paroi (W) entre une zone supérieure (T) et une zone inférieure (B) de la forme d'onde est inférieure à une densité de fibre dans l'une quelconque parmi la zone supérieure (T) et la zone inférieure (B) ;
**caractérisé en ce que** :
les fibres (24) constituant la feuille de surface (21) sont plus orientées dans une direction longitudinale de la feuille de surface (21) que dans sa direction transversale ;
la forme d'onde est formée de manière continue dans la direction longitudinale dans des régions de formage de forme multiple (R1) de la feuille de surface (21), les régions de formage de forme multiple (R1) s'étendant chacune dans la direction longitudinale et étant alignées dans la direction croisant la direction longitudinale ;
une région de formage sans forme (R2) est formée entre toutes les deux régions adjacentes des régions de formage de forme (R1), les régions de formage sans forme (R2) s'étendant également dans la direction longitudinale ; et
les régions de formage de forme (R1) sont positionnées dans une position déplacée vers le côté d'une région de ceinture arrière.

2. Article absorbant selon la revendication 1, dans lequel les fibres (24) dans la zone supérieure (T) et la zone inférieure (B) sont liées par fusion.

3. Article absorbant selon l'une quelconque des revendications 1 et 2, dans lequel
la densité de fibre de chacune parmi la zone supérieure (T) et la zone inférieure (B) est dans une plage de 0,05 [g/cm³] à 0,2 [g/cm³] inclus, et
la densité de fibre de la zone de paroi (W) est dans une plage de 0,005 [g/cm³] à 0,15 [g/cm³] inclus.

4. Article absorbant selon l'une quelconque des revendications 1 à 3, dans lequel
la zone supérieure (T) et la zone inférieure (B) ont chacune des dimensions dans les plages de 0,2 [mm] à 3 [mm] inclus dans la direction longitudinale et de 3 [mm] à 100 [mm] inclus dans la direction transversale,
la zone de paroi (W) a des dimensions dans les plages de 0,3 [mm] à 5 [mm] y compris dans la direction longitudinale et de 3 [mm] à 100 [mm] inclus dans la direction transversale, et
une dimension de hauteur de la forme d'onde de la partie inférieure à la partie supérieure est dans une plage de 0,3 [mm] à 3 [mm] inclus.

5. Article absorbant selon l'une quelconque des revendications 1 à 4, dans lequel les fibres (24) dans la zone de paroi (W) sont étirées, moyennant quoi un diamètre de fibre dans la zone de paroi (W) est inférieur aux diamètres de fibre dans la zone supérieure (T) et la zone inférieure (B).

6. Article absorbant selon l'une quelconque des revendications 1 à 5, dans lequel dans la zone de paroi (W), les fibres (24) ou les parties de liaison des fibres (24) sont coupées.

7. Article absorbant selon l'une quelconque des revendications 1 à 6, dans lequel la feuille de surface (21) comprend une région de formage sans forme (R2) s'étendant dans la direction longitudinale et ayant une dimension de direction transversale de 0,2 [mm] à 3 [mm] inclus.

8. Article absorbant selon l'une quelconque des revendications 1 à 7, comprenant un tissu non tissé qui est formé sur une surface, au niveau d'un côté absorbant, de la région de formage de forme (R1) dans la feuille de surface (21) et est plus volumineux que la feuille de surface (21).

9. Article absorbant selon la revendication 8, dans lequel le tissu non tissé volumineux a une densité dans une plage de 0,05 [g/cm³] à 0,10 [g/cm³] inclus.

10. Article absorbant selon l'une quelconque des revendications 8 à 9, comprenant une feuille qui est disposée entre le tissu non tissé volumineux et l'absorbant (23) et recouvre l'absorbant (23), dans lequel la feuille a une densité dans une plage de 0,05 [g/cm³] à 0,25 [g/cm³] inclus.
